# EUROPEAN PATENT SPECIFICATION

(11) **EP 0 979 117 B1**
(45) Date of publication and mention of the grant of the patent: **26.10.2005**
(21) Application number: 98914270.8
(22) Date of filing: 03.04.1998
(51) Int. Cl.: A61M 16/06

(54) **NASAL CANNULA**
NASENKANÜLE
CANULE NASALE

(30) Priority: 29.04.1997 US 45080 P
(43) Date of publication of application: 16.02.2000
(73) Proprietor: Salters Labs, Arvin, CA 93203 (US)
(72) Inventor: CURTI, James, Noah, Bakersfield, CA 93309 (US); CHUA, James, Tehachapi, CA 93561 (US); SALTER, Peter, W., Tehachapi, CA 93561 (US)
(74) Representative: Watts, Peter Graham
(86) International application number: PCT/US1998/005573
(87) International publication number: WO 1998/048876

(56) References cited:
- FR-A- 2 622 115
- US-A- 4 660 555
- US-A- 4 753 233
- US-A- 4 958 075
- US-A- 5 046 491
- US-A- 5 137 017
- US-A- 5 335 656
- US-A- 5 400 781
- US-A- 5 477 852
- US-A- 5 664 567

## Description

### BACKGROUND OF THE INVENTION

The practice of measuring end-tidal carbon dioxide during the administration of anesthesia, particularly regional anesthesia, has grown markedly in the past several years. The reasons that anesthesiologists have embraced this technique are described more fully in U.S. Patent No. 5,335,656.

US-patent No. 5,046,491 discloses the features of the preamble of claim 1.

The preferred nasal cannula used in this procedure is a cannula which insufflates the patient with oxygen through one nare of a cannula and separately samples the exhaled gases by drawing the exhaled gas from the other nare into a conventional carbon dioxide analyzer. The cannula is preferably provided with an internal wall or system in the face piece to keep the conduits separate for insufflation and sampling, however, separate lines can be used or even multiple nares for insufflation and sampling, though the latter device substantially increases the risk of gases mixing which can distort the readings for end-tidal carbon dioxide. It is preferred that two nares only are employed and that each nare performs only one function, i.e., insufflation or sampling into or from separate nostrils. Likewise, insufflation has normally been continuous, however, it could advantageously be intermittent which would further improve the end-tidal carbon dioxide measurement by insuring that gases being sampled were representative of exhaled gases undiluted by the other gases being insufflated. Most preferably, the intermittent insufflation is accomplished by the apparatus and method described in U. S. Patent No. 5,626,131. Other so-called demand insufflation devices which begin insufflation upon the start of inhalation can also be employed.

Normal nasal cannulae are designed with the nares having a slight inward curvature as they extend upward from the face piece. This is anatomically desirable and important for imparting the proper direction of insufflating gas into the nasal cavities. When the patient is in the upright sitting position or ambulatory, this is the most satisfactory design configuration. Conversely, problems can be encountered if the patient is horizontal or prone and tends to accumulate secretions in the nasal cavities. It can be a particularly vexing problem if either the insufflation or sampling nare becomes occluded during the use of the cannula for sampling and monitoring end-tidal carbon dioxide during the administration of anesthesia.

### OBJECTS OF THE INVENTION

It is therefore an object of the present invention to provide a nasal cannula structure for sampling carbon dioxide which reduces or eliminates the incidence of occlusion of the tip of the carbon dioxide sampling nare during the removal of carbon dioxide by the sampling line connected to a monitoring device and/or a source of suction or vacuum.

It is also an object of the present invention to provide a nasal cannula for insufflating a patient with oxygen while accurately monitoring end-tidal carbon dioxide, that will continue to function properly for its intended purpose when either or both nares become occluded for any reason.

It is a further object to accomplish the foregoing objects with a minimum risk of distorting the end-tidal carbon dioxide readings from the sampled exhalation gases during the administration of anesthesia.

### BRIEF SUMMARY OF THE INVENTION

The foregoing objects and advantages are obtained by providing a nasal cannula structure according to claim 1, that is adapted for insufflation and sampling, with additional holes or vents on the nares of the nasal cannula, preferably both anterior and posterior of one or both nares at a location proximate the entrance of the nasal passageways when the cannula is in use.

### BRIEF DESCRIPTION OF THE DRAWINGS

Figure 1 is a frontal view of a normally positioned nasal cannula on a patient (shown in phantom) connected to a gas source (G) and a gas analyzer (A).
Figure 2 is a rear view of the cannulae face piece shown in Figure 1.
Figure 3 is a partial cross section of a nare of the nasal cannula taken along the lines and arrows 3-3 of Figure 2.
Figure 4 is a plan view of the nasal cannula of Figure 2.

### DETAILED DESCRIPTION OF THE ILLUSTRATED EMBODIMENT

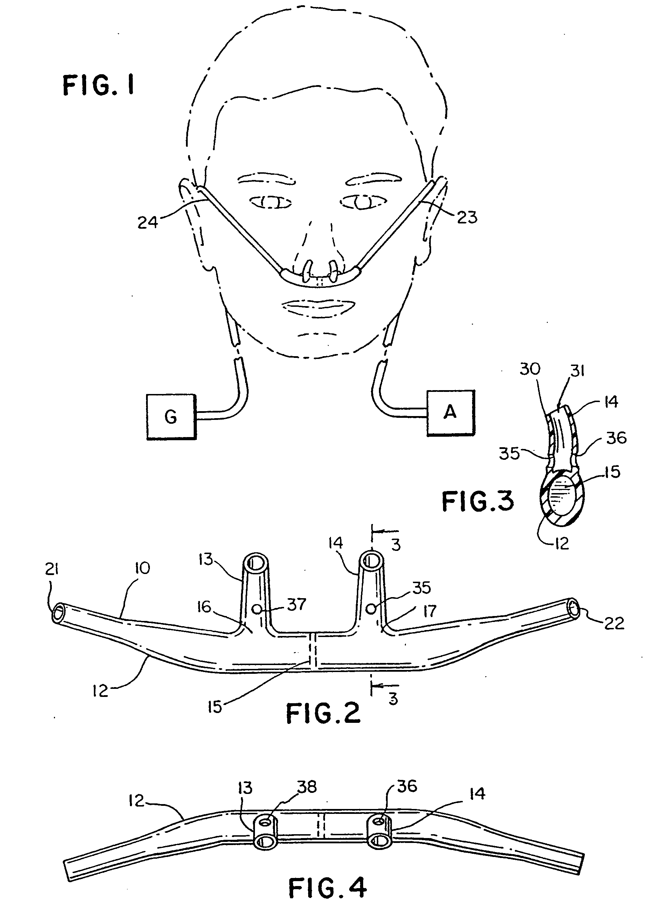

The nasal cannula 10 of one embodiment of the present invention consists of a generally tubular face piece 12 having two nares 13 and 14 and a septum 15 disposed in the center of the face piece 12 between the openings 16 and 17, respectively, of the nares 13 and 14 (see Figs. 2, 3 and 4). The openings 21 and 22 on the ends of the face piece 12 are affixed to separate tubes 23 and 24 as shown in Fig. 1, which are separately connected to a source of insufflating gas (G), such as oxygen, and a commercial carbon dioxide monitoring unit (shown as A) which, in turn, has or is connected to a vacuum pump or other means for drawing exhaled breath containing carbon dioxide into an instrument that is capable of measuring the concentration of the carbon dioxide in the sampled gas.

During use of the cannula for both insufflation and the monitoring of carbon dioxide concentration in the exhaled breath (depicted schematically in Fig. 1), the readings for end-tidal carbon dioxide can become distorted where there is undesirable mixing with room air or with excess insufflating gas. Likewise, carbon dioxide measuring devices which typically employ varying amounts of suction or vacuum to obtain the gas sample to be analyzed, can unduly dilute the sample or more seriously can draw the tip 30 of the sampling nare (representatively shown in Fig. 3) onto the adjacent surface of the tissue of the nasal passage and occlude the opening 31 thereby restricting or even preventing sampling of the exhaled gases for their carbon dioxide concentration.

This is an especially serious problem where the patient is prone and secretions can be present which are drawn into the opening 31 at the tip 30 and which then either partially or totally occlude the opening 31, during the administration of anesthesia.

The anesthesiologist must respond by clearing the nare opening after first removing the cannula from its location on the face of the patient. This may be complicated where the patient is draped in a manner which covers the cannula, such as in eye surgery. It may also be difficult to detect the occlusion where the end-tidal carbon dioxide measurement signal is only partially degraded.

It has been discovered that the expedient of additionally providing the nares with very small holes, shown collectively at 35 and 36 and 37 and 38, achieves the desired result of preventing an undesirable and unnecessary level of suction at the opening 31 of the tip 30 from developing sufficiently to draw the opening 31 into the nasal tissue thereby occluding the opening. The holes are sized large enough to prevent sufficient suction from developing at the tip 30 to draw in mucosal secretions or attach the tip by suction to the soft mucosal tissue, while still drawing an undiluted sample of the exhaled gases to provide good end-tidal carbon dioxide measurements. Likewise, too large an opening for these holes would undesirably dilute the exhaled gas sample with room air or excess insufflation gas.

Most preferably, as previously noted, the nasal cannula of the present invention can be used in combination with an oxygen delivery system that delivers the insufflating gas intermittently. The delivery can be initiated at any time after the peak end-tidal carbon dioxide measurement is achieved during exhalation and continuing into the inhalation phase of the breathing cycle or could be inhalation activated or designed to deliver only during selected portions of all or only some of the inhalation phases of a patient's breathing cycles. Preferably, the delivery should begin before the termination of the exhalation phase, such as is described in U.S. Patent No. 5,626,131. Using intermittent delivery substantially reduces the possibility of distorted carbon dioxide readings due to gas mixing.

Likewise, slits or slots (not shown) may be employed in the nares which could function in the same manner as the holes described if they are positioned in such a manner to avoid collapse or occlusion with the nasal tissues and provide the desired function of preventing sufficient suction from developing at the tip of the nare to cause it to be drawn, by suction, onto the tissues. The holes provided as described herein are preferred as there is less risk of occlusion and trauma from the edges of slits or slots to the nasal tissue and potentially there is less risk of gas dilution and mixing from occurring where the slits or slots are overly large.

Further, the combination of intermittent insufflation using the cannula of the present invention produces the desired end-tidal carbon dioxide measurement, as described, and helps prevent patient desaturation during the rigors of surgery and anesthesia administration.

Preferably, the size of the openings is from between about 1.27mm to about 1.78mm (about 0.05 to about 0.07 inches) though larger or smaller holes or single holes may be advantageously employed in combination with specific analytical apparatuses. The size and location of the openings can vary with the analyzer selected and the proper function confirmed without undue experimentation.

The invention described herein is to be limited only by the scope of the appended claims.

## Claims

1. An apparatus for insufflating a treating gas into the nose of a patient and measuring carbon dioxide content in the exhalation of the patient, said apparatus consisting of:
an elongated hollow body (10) including a tubular portion (12) adapted to be received on the skin surface adjacent the nose of the patient;
a wall (15) within said hollow body (12) defining therein both an inhalation manifold and an exhalation manifold, said wall (15) providing a gas-tight seal positively preventing fluid communication between said inhalation and exhalation manifolds;
a supply of treating gas (G) connected to said inhalation manifold;
a first hollow prong (13) attached to the hollow body (12) and opening at a first end (16) into said inhalation manifold and having an opening (13) at a second free end; said first hollow prong (13) being adapted to be received in a first nasal passage of the nose of the patient, whereby treating gas may be insufflated into the first nasal passage through the opening (31);
a second hollow prong (14) attached to the hollow body (12) and opening at a first end (17) into said exhalation manifold and having an opening (31) at a second free end, said second hollow prong (14) being adapted to be received in a second nasal passage of the nose of the patient, whereby a portion of the exhalation from the patient may be withdrawn from the second nasal passage through the opening (31);
said first and second prongs (13,14) each being substantially smaller in diameter than the respective nasal passages, so as not to occlude said passages;
said second prong (14) being provided with at least one additional opening (35,36) communicating with the hollow interior of said second prong (14); and
means (A) for measuring the concentration of carbon monoxide in the exhaled gases being connected to said exhalation manifold, said means (A) including means for withdrawing an exhaled gas sample from said exhalation manifold;
**characterised in that**; said additional opening (35,36) is located proximate the entrance of the second nasal passage, when the second prong (14) is received in the second nasal passage; and
said additional opening (35,36) has a diameter from 1.27 to 1.78 mm (0.05 to 0.07 inches).

2. An apparatus according to claim 1 **characterised in that** an additional opening is provided in the first hollow prong (13), said additional opening (37,38) being located proximate the entrance of the first nasal passage, when the first prong (13) is received in the first nasal passage and said additional opening (37,38) having a diameter from 1.27 to 1.78 mm (0.05 to 0.07 inches).

3. An apparatus according to claim 1 or 2 **characterised in that** a pair of additional openings are aligned diametrically of one another in the first and/or second prong (13,14).

4. An apparatus according to any one of claims 1 to 3 **characterised in that** the or each additional opening (35,36,37,38) in the first and second prongs (13,14) is located closer to the attachment of the first/second prong (13,14) to the hollow body (12) than to the free end of the first/second prong (13,14).

5. An apparatus according to any one of claims 1 to 4 **characterised in that** the or each additional opening (35,36,37,38) in the first and second prongs (13,14) is located adjacent the attachment of the first/second prong (13,14) to the hollow body (12).

6. An apparatus according to any one of the preceding claims **characterised in that** the first and/or second prong (13,14) has a constantly tapering outer diameter which tapers from the end (16,17) attached to the elongate hollow body (12) to the free end of the prong (13,14).

## Patentansprüche

1. Vorrichtung zum Einblasen eines Behandlungsgases in die Nase eines Patienten und Messen des Kohlendioxidgehaltes in der Ausatmung des Patienten, wobei die Vorrichtung aus folgendem besteht:
einem länglichen Hohlkörper (10), der einen rohrförmigen Teil (12) enthält, der ausgebildet ist, um auf der Hautoberfläche angrenzend an die Nase des Patienten aufgenommen zu werden;
einer wand (15) innerhalb des Hohlkörpers (12), die darin sowohl einen Einatmungeverteiler als auch einen Ausatmungsverteiler begrenzt, wobei die Wand (15) eine gasdichte Dichtung schafft, die eine Fluidverbindung zwischen dem Einatmungs- und Ausatmungsverteiler ausdrücklich verhindert;
einer Behandlungsgas (G)-Versorgung, die mit dem Einatmungsverteiler verbunden ist;
einer ersten Hohlzinke (13), die an dem Hohlkörper (12) befestigt ist und an einem ersten Ende (16) in den Einatmungsverteiler mündet und eine Öffnung (13) an einem zweiten freien Ende hat; wobei die erste Hohlzinke (13) ausgebildet ist, um in einem ersten Nasenkanal der Nase des Patienten aufgenommen zu werden, wodurch Behandlungsgas in den ersten Nasenkanal durch die Öffnung (31) hindurch eingeblasen werden kann;
einer zweiten Hohlzinke (14), die an dem Hohlkörper (12) befestigt ist und an einem ersten Ende (17) in den Ausatmungsverteiler mündet und eine Öffnung (31) an einem zweiten freien Ende hat, wobei die zweite Hohlzinke (14) ausgebildet ist, um in einem zweiten Nasenkanal der Nase des Patienten aufgenommen zu werden, wodurch ein Teil der Ausatmung von dem Patienten von dem zweiten Nasenkanal durch die Öffnung (31) hindurch abgeführt werden kann;
wobei jede der aus der ersten und zweiten Zinke (13, 14) bestehenden Zinken im Durchmesser kleiner als die jeweiligen Nasenkanäle ist, um diese Kanäle nicht zu verstopfen;
wobei die zweite Zinke (14) mit mindestens einer weiteren Öffnung (35, 36) versehen ist, die mit dem Hohlraum im Inneren der zweiten Zinke (14) in Verbindung steht und
eine Einrichtung (A) zum Messen der Konzentration von Kohlenmonoxid in den ausgeatmeten Gasen mit dem Ausatmungsverteiler verbunden ist, wobei die Einrichtung (A) ein Mittel zum Abführen einer Probe von ausgeatmetem Gas aus dem Ausatmungsverteiler enthält;
**dadurch gekennzeichnet, daß** die weitere Öffnung (15, 36) in der Nähe des Einganges des zweiten Nasenkanals angeordnet ist, wenn die zweite zinke (14) in dem zweiten Nasenkanal aufgenommen ist; und
die weitere Öffnung (35, 36) einen Durchmesser von 1,27 bis 1,78 mm (0,05 bis 0,07 Zoll) hat.

2. Vorrichtung nach Anspruch 1, **dadurch gekennzeichnet, dass** eine weitere Öffnung in der ersten Hohlzinke (13) vorgesehen ist, wobei die weitere Öffnung (37, 38) in der Nähe des Einganges des ersten Nasenkanals angeordnet ist, wenn die erste Zinke (13) in dem ersten Nasenkanal aufgenommen ist und wobei die weitere Öffnung (37, 38) einen Durchmesser von 1,27 bis 1,78 mm (0,05 bis 0,07 Zoll) hat.

3. Vorrichtung nach Anspruch 1 oder 2, **dadurch gekennzeichnet, dass** zwei weitere Öffnungen diametral zueinander in der ersten und/oder zweiten Zinke (13, 14) angeordnet sind.

4. Vorrichtung nach einem der Ansprüche 1 bis 3, **dadurch gekennzeichnet, dass** die oder jede weitere Öffnung (35, 36, 37, 38) in der ersten und zweiten Zinke (13, 14) näher an der Befestigung der ersten/zweiten Zinke (13, 14) an dem Hohlkörper (12) als an dem freien Ende der ersten/zweiten Zinke (13, 14) angeordnet ist.

5. Vorrichtung nach irgendeinem der Ansprüche 1 bis 4, **dadurch gekennzeichnet, dass** die oder jede weitere Öffnung (35, 36, 37, 38) in der ersten und zweiten Zinke (13, 14) angrenzend an die Befestigung der ersten/zweiten Zinke (13, 14) an dem Hohlkörper (12) angeordnet ist.

6. Vorrichtung nach irgendeinem der vorhergehenden Ansprüche, **dadurch gekennzeichnet, dass** die erste und/oder zweite Zinke (13, 14) einen sich stetig verjüngenden Außendurchmesser hat, der sich von dem an dem länglichen Hohlkörper (12) befestigten Ende (16, 17) zu dem freien Ende der Zinke (13, 14) hin verjüngt.

## Revendications

1. Un appareil pour insuffler un gaz de traitement dans le nez d'un patient et mesurer le contenu en dioxyde de carbone de l'exhalation du patient, ledit appareil comprenant :
un corps creux allongé (10) possédant une portion tubulaire (12) adaptée pour être reçue sur la surface cutanée adjacente au nez du patient ;
une paroi (15) à l'intérieur dudit corps creux (12) définissant à la fois un collecteur d'inhalation et un collecteur d'exhalation, ladite paroi (15) fournissant un joint étanche au gaz empêchant positivement la communication de fluide entre lesdits collecteurs d'inhalation et d'exhalation ;
un approvisionnement en gaz de traitement (G) connecté audit collecteur d'inhalation ;
une première branche ou fourche creuse (13) attachée au corps creux (12) débouchant à une première extrémité (16) à l'intérieur dudit collecteur d'inhalation et possédant une ouverture (31) à une deuxième extrémité libre; ladite première branche creuse (13) étant adaptée pour être reçue dans un premier passage nasal du nez du patient, le gaz de traitement pouvant ainsi être insufflé dans le premier passage nasal à travers l'ouverture (31) ;
une deuxième branche ou fourche creuse (14) attachée au corps creux (12) débouchant à une deuxième extrémité (17) dans ledit collecteur d'exhalation et possédant une ouverture (31) à une deuxième extrémité libre, ladite deuxième branche creuse (14) étant adaptée pour être reçue dans un deuxième passage nasal du nez du patient, une portion de l'exhalation du patient pouvant ainsi être soutirée du deuxième passage nasal à travers l'ouverture (31) ;
le diamètre de chacune desdites première et deuxième branches (13, 14) étant substantiellement plus réduit que les passages nasaux respectifs, de manière à ne pas obstruer lesdits passages ;
ladite deuxième branche (14) étant fournie avec au moins une ouverture supplémentaire (35, 36) communiquant avec l'intérieur creux de ladite branche (14) ; et
un dispositif (A) pour mesurer la concentration de monoxyde de carbone dans les gaz exhalés étant couplé avec ledit collecteur d'exhalation, ledit dispositif (A) comprenant des moyens pour soutirer un échantillon de gaz exhalés dudit collecteur d'exhalation ;
**caractérisé en ce que** : ladite ouverture supplémentaire (35, 36) est située à proximité de l'entrée du deuxième passage nasal lorsque la deuxième branche (14) est reçue dans le deuxième passage nasal ; et
ladite ouverture supplémentaire (35, 36) possède un diamètre de 1,27 à 1,7 mm (0,05 à 0,07 pouce).

2. Un appareil selon la revendication 1, **caractérisé en ce qu'**une ouverture supplémentaire (37, 38) est fournie dans la première branche creuse (13), ladite ouverture supplémentaire (37, 38) étant située à proximité de l'entrée du premier passage nasal lorsque la première branche (13) est reçue dans le premier passage nasal, et ladite deuxième ouverture (37, 38) possédant un diamètre 1,27 à 1,78 mm (0,05 à 0,07 pouce).

3. Un appareil selon la revendication 1 ou 2, **caractérisé en ce qu'**une paire d'ouvertures supplémentaires sont alignées diamétralement l'une par rapport à l'autre dans la première et/ou la deuxième branche (13, 14).

4. Un appareil selon l'une quelconque des revendication 1 à 3, **caractérisé en ce que** la ou chaque ouverture supplémentaire (35, 36, 37, 38) dans les première et deuxième branches (13, 14) est située plus proche de l'attachement de la première/deuxième branche (13, 14) au corps creux (12) qu'elle ne l'est de l'extrémité libre de la première/deuxième branche (13, 14).

5. Un appareil selon l'une quelconque des revendication 1 à 4, **caractérisé en ce que** la ou chaque ouverture supplémentaire (35, 36, 37, 38) dans les première et deuxième branches (13, 14) est située adjacente à l'attachement de la première/deuxième branche (13, 14) au corps creux (12).

6. Un appareil selon l'une quelconque des revendication précédentes, **caractérisé en ce que** la première et/ou la deuxième branche (13, 14) possède un diamètre extérieur à conicité constante, dont le diamètre décroît de l'extrémité (16, 17) attachée au corps creux allongé (12) vers l'extrémité libre de la branche (13, 14).
